**Europäisches Patentamt**

**European Patent Office**

**Office européen des brevets**

(19)

(11) Veröffentlichungsnummer : **0 331 152 B1**

(12) # EUROPÄISCHE PATENTSCHRIFT

(45) Veröffentlichungstag der Patentschrift :
09.10.91 Patentblatt 91/41

(51) Int. Cl.⁵ : **A61K 9/00, A61K 35/16, A61K 37/54, A61M 5/178**

(21) Anmeldenummer : **89103600.6**

(22) Anmeldetag : **01.03.89**

(54) Zweikammerspritze mit einer Füllung aus aktivitätsempfindlichem humanen Protein als Wirkstoff.

(30) Priorität : 01.03.88 DE 3806562

(43) Veröffentlichungstag der Anmeldung :
06.09.89 Patentblatt 89/36

(45) Bekanntmachung des Hinweises auf die
Patenterteilung :
09.10.91 Patentblatt 91/41

(84) Benannte Vertragsstaaten :
AT BE CH DE ES FR GB GR IT LI LU NL SE

(56) Entgegenhaltungen :
EP-A- 0 211 592
EP-A- 0 221 566
WO-A-86/01120
US-A- 3 756 390

(73) Patentinhaber : **ALPHA THERAPEUTIC GMBH**
**Voltastrasse 10**
**W-6070 Langen (DE)**

(72) Erfinder : **Dittmann, Otto**
**Am Haufensteinberg 14**
**W-6101 Brensbach-Wallbach (DE)**

(74) Vertreter : **Patentanwälte Deufel, Hertel, Lewald**
**Isartorplatz 6 Postfach 26 02 47**
**W-8000 München 26 (DE)**

**Beschreibung**

Es werden seit langer Zeit Zweikammerspritzen verwendet, wobei in einer Kammer pyrogenfreies Lösungsmittel und in der zweiten Kammer, die der Spritzennadel zugekehrt ist, eine durch Lyophylisieren in der Spritze selbst erhaltene Füllung vorliegt, die bei Betätigung der Spritze durch das pyrogenfreie Wasser gelöst und dann injiziert wird.

Nun gibt es eine ganze Reihe von Wirkstoffen auf der Basis humaner Plasma-Proteine, die aktivitätsempfindlich sind. Dies bedeutet, daß sie nicht in höherer Konzentration über vernünftige Zeiträume von beispielsweise einigen Stunden in Lösung gehalten werden können, da die Substanz dabei biologische Aktivität verliert oder gar zersetzt werden kann und zum Teil ausfällt. Dies gilt insbesondere für Faktor VIII. Derartige Präparate können derzeit nur in verhältnismäßig geringer Konzentration verwendet werden. Für hinreichend therapeutisch wirksame Injektionslösungen ist die Konzentration in den für Injektionsspritzen noch tragbaren Ampullengrößen zu gering. Sie werden daher als Infusionslösung eingesetzt. Da diese Lösungen notwendigerweise sehr verdünnt sein müssen, dauert die Infusion und somit die Zufuhr des Wirkstoffes sehr lange. Erwünscht ist aber in den meisten Fällen eine schnelle Zufuhr der Wirkstoffe, um eine möglichst schnelle Wirkung zu erzielen. Selbst für mäßig erhöhte Konzentrationen solcher Wirkstoffe ist aber die für eine Infusion erforderliche Zeitspanne häufig zu groß, um eine Zersetzung oder Verschlechterung der Substanz auszuschließen.

Für den Einsatz zur direkten Lyophilisierung in der Zweikammerspritze waren die bisher erhältlichen Lösungskonzentrationen zu gering, um bei einer einmaligen Lyophylisierung eine hinreichende Menge der Substanz in der Kammer zu erhalten. Ein mehrmaliges Auffüllen und Lyophilisieren in der Kammer ist aber aus den gleichen Gründen nicht möglich, die für den Einsatz höher konzentrierter Lösungen allgemein gelten, da wegen der Aktivitätsempfindlichkeit solcher humaner Proteine beim erneuten Füllen der Kammer das Protein geschädigt werden könnte.

Es ist nun gelungen, wesentlich höhere Lösungskonzentrationen solcher Proteine zu erzielen, die hinsichtlich der zeitlichen Beständigkeit und der Konzentration ausreichen, um bei einmaliger Befüllung der Spritzenkammer und sofortiger Lyophilisierung den Wirkstoff ohne Schädigung in die Spritzenkammer zu bringen, so daß eine so präparierte Spritze längere Zeit gelagert werden kann und erst beim Einsatz unmittelbar vor der Injektion das Protein durch das pyrogenfreie Lösungsmittel in der anderen Spritzenkammer gelöst und sofort injiziert wird. Somit beträgt die Zeit in Lösung vor der tatsächlichen Anwendung höchstens einige Minuten.

Auf diese Weise ist es nun möglich, aktivitätsempfindliche humane Proteine in der gewünschten hohen Konzentration, also innerhalb einer kurzen Zeitspanne zu verabreichen.

Solche Präparate sind insbesondere Urokinase, Pro-Urokinase, TPA, Streptokinase, Faktor VIII und Faktor IX sowie Antithrombin III.

So kann man bei den üblichen Kammergrößen solcher Spritzen von bis zu 6 ml der Kammer, welche die zu Lyophilisierende Lösung (also den Wirkstoff) aufweist und bis zu 4,5 ml für das Lösungsmittel beim Einfüllen von 3 bis 3,5 cm³ des Plasmakonzentrates etwa mindestens 2 bis 3 Millionen Einheiten Urokinase, Pro-Unrokinase, TPA oder Streptokinase, mindestens ca. 1000 Einheiten Faktor VIII oder Faktor IX und mindestens ca. 500 bis 1000 Einheiten Antithrombin III bei einmaligem Lyophilisieren in der Wirkstoffkammer der Spritze unterbringen.

Man kann bei einem Kammervolumen von 6 ml höchstens 3 bis 3,5 cm³ an zu lyophilisierender Lösung einfüllen, da beim Lyophilisieren die Lösung spritzt und an der Wand hochkriecht. Dies unterstreicht die Wichtigkeit relativ hoch konzentrierte Lösungen für das Befüllen zu verwenden, um therapeutisch brauchbare Mengen in der Spritzenkammer unterzubringen.

Eine solche Spritze sei nun näher beschrieben.

Eine bekannte Zweikammerspritze weist in der der Kanüle zunächstliegenden vorderen bzw. zweiten Kammer den lyophilisierten Wirkstoff auf. Links am Hals dieser Kammer sitzt die getrennte Verschlußkappe, auf welche die Kanüle aufgesetzt wird. Zwischen den Kammern ist eine mit kleinen Löchern versehene Platte, welche durch die Löcher und die durch eine Ausbuchtung der Spritzenwand gebildete Erhebung beim Eindrücken des Kolbens in die Lösungsmittelkammer das enthaltene Lösungsmittel in die vordere Kammer drückt, wo man durch kurzes Schütteln die Auflösung des lyophilisierten Wirkstoffes bewirkt und dann sofort den Inhalt der Spritze verabreichen kann.

Da man nicht beliebig große Mengen an Injektionslösung injizieren kann und selbst bei i.v.-Injektion die auf einmal zu injizierende Menge nicht zu groß sein sollte, kann ein Volumen der Kammer 1 von 6 ml kaum überschritten werden. Aus diesem Grunde ist es wesentlich, in einer Kammer bis zu dieser Größe möglichst viel Wirkstoff durch einmaliges Lyophilisieren, also auf einmal einzubringen.

Das Befüllen der Spritze erfolgt in an sich bekannter Weise indem, wie dies auch für das Befüllen von Fertigspritzen üblich ist, unter sterilen Bedingungen die vorgegebene Menge der Lösung des einzubringenden aktivitätsempfindlichen humanen Proteins eingebracht wird, worauf die Spritze zur nächsten Station gebracht wird, wo in situ lyophilisiert wird. Die Spritze mit dem gefriergetrockneten Inhalt wird dann zur nächsten Station gebracht, wo das pyrogenfreie Lösungsmittel in die zweite Kammer eingefüllt wird, worauf dann unter sterilen Bedingungen der endgültige Zusammenbau und die Verpackung erfolgen. Man kann dazu die für das Befüllen von Fertigspritzen üblichen Automaten mit nur geringfügigen Umbauten, um die Spritzengröße und das Einsetzen des Kolbens und die von der üblichen Stopfenform abweichende Form der Verschlußkappe der Spritze zu berücksichtigen, verwenden.

Solche Spritzen eignen sich nicht nur für die Anwendung in z.B. der ärztlichen Praxis und im Klinikbereich sondern insbesondere auch in der Unfallmedizin und zur Heimselbstbehandlung durch den Patienten.

Das Aufkonzentrieren der Lösungen der aktivitätsempfindlichen humanen Proteine soll anhand einiger Beispiele gezeigt werden. Auf diese Weise kann eine erheblich höhere Lösungskonzentration erreicht wird, als dies bis jetzt möglich war, so daß man bei einem einzigen Lyophylisiergang eine Spritzenkammer von z.B. 6 ml Inhalt, die für das Lyophylisieren mit höchstens 3,5 ml gefüllt werden kann, damit kein Überspritzen und Hochkriechen beim Lyophylisieren erfolgt, mit der ausreichenden Menge an Protein versieht, um mit einer einzigen Spritzenfüllung die für die wirksame Behandlung ausreichende Menge an Protein bereitzustellen.

Die folgenden Beispiele erläutern die Erfindung.

Beispiel 1

Herstellung von konzentrierter Urokinaselösung

Ausgehend von ca. 1500 l Urin wird in einer größeren Anzahl von Ansätzen, z.B. ca. 50, ein Bentonit-Aktivator-Komplex hergestellt, aus dem wiederum die rohe Urokinase in an sich bekannter Weise gewonnen wird. Aus dieser wird in mehreren Ansätzen ein Calciumphosphat-Urokinase-Komplex gebildet, aus dem wiederum in zwei Ansätzen gereinigte konzentrierte Urokinase mit 500.000 bis 600.000 I.E./ml, enthalten in einer Menge von 5-10 l gewonnen wird.

Im einzelnen ist die Arbeitsweise wie folgt :

Der mit Borsäure konzentrierte Urin wird mit Bentonit versetzt, der Bentonit-Aktivator-Komplex wird mit Wasser für Injektionszwecke gewaschen, mit einer Lösung von 120 kg Ammoniumsulfat, 6,2 kg eines nichtionogenen Ätherglykols in Form eines Blockpolymeren, der unter der Handelsbezeichnung Pluronic erhältlich ist, mit Wasser aufgefüllt auf 310 l, eluiert und das Eluat mit ca. 165 kg Ammoniumsulfat ausgesalzen. Die erhaltene rohe Urokinase-Bulkware wird mit einer Lösung von 0,9 kg Ethacridinlactat in 430 l Wasser für Injektionszwecke eluiert und das Eluat an Calciumphosphat-Gel, das aus 11 kg Natriumphosphat × 12 H$_2$O, 6,4 kg Calciumchlorid × 2H$_2$O aufgefüllt, mit Wasser für Injektionszwecke auf 180 l aufgefüllt, gewonnen ist, absorbiert. Der Calcium-Urokinase-Komplex wird mit einer Lösung von ca. 12,5 kg Natriumdihydrogenphosphat × 2H$_2$O aufgefüllt auf 220 l, mit 3-N Natronlauge, eluiert und dann das Eluat an CM-Cellulose bzw. CM-Sephadex gereinigt. Der so erhaltene CM-Cellulose-Urokinase-Komplex wird mit einer Lösung von 50 kg Natriumdihydrogenphsophat × 2H$_2$O aufgefüllt auf 165 l mit 3-N Natronlauge eluiert, das Eluat mit Phosphorsäure angesäuert, ca. 10 h bei 60°C pasteurisiert und dann gegen 0,1 M-Posphat Pufferlösung vom pH 7,1 dialysiert und filtriert. Das Dialysat wird dann nach den üblichen Standardmethoden für gereinigtes Urokinasekonzentrat geprüft. Die Urokinasekonzentration liegt gewöhnlich zwischen 70000 und 160000 I.E./ml und die Phosphatkonzentrationen sind die gleichen wie bei 0,1 M Phosphat Pufferlösung zur Dialyse. Gegebenenfalls wird die Urokinaselösung auf die gewünschte Konzentration verdünnt, indem man die geeignete Menge an Albumin-Phosphat-Pufferlösung von pH 7,1, in welcher das NaH$_2$PO$_4$ · 2H$_2$O/Na$_2$HPO$_4$ · 12H$_2$O-Verhältnis 13/60 Gew.-Teile beträgt, zum Dialysat zufügt. Die Lösung der gewünschten Konzentration wird dann in die Spritzen gefüllt und darin lyophilisiert.

Die folgenden Ionenaustauscher und Membranfilter werden bei der Herstellungsweise benutzt.

Als Ionenaustauscher Carboxymethylcellulose wird eine Ionenaustauscherkapazität von 1,14 mEq/g Trockengewicht, CM-Sepahdex, Typ C-25 mit einer Ionenaustauscherkapazität von 4,5 +/– 0,5 mEq/g Trockengewicht und als Membranfilter eine Polyamidfilter einerseits von der Porengröße 0,2 µm und andererseits 0,8 µm.

Beispiel 2

Herstellung einer konzentrierten Antithrombin -III-Lösung

Die Isolierung von Antithrombin aus Blutplasma und Blutprodukten, insbesondere aus der Cohn-Plasmafraktion-VI-1-Paste erfolgt in an sich bekannter Weise durch Reinigung mittels Polyethylenglykol-Fraktionierung, Heparin-Sepharose 4B-Affinitäts-Chromatographie und Wärmebehandlung bei 60°C für 10 h. Wesentlich dabei ist, daß bei der Affinitäts-Chromatographie nicht eine Graduenteneluierung mit Hilfe eines Puffers mit zunehmender NaCl-Konzentration erfolgt, sondern die Konzentration des Wasch- und Eluierungsmittels, nämlich 0,4 M-NaClO, 02 M-Imidazollösung von ph 7,3 für das Waschen der Säule und für die Elution 2 M-NaClO, 02 M-Imidazollösung von pH 6,5 immer gleichbleibt. Die erhaltene Antithrombin-III Lösung wird an Hohlfasern konzentriert mit Natriumzitrat versetzt und gerührt und bei 60°C 10 h pasteurisiert. Dann werden die Salze durch Dialyse an Hohlfasern entfernt. (Lösungsmittel für die Dialyse : Na$_3$C$_6$H$_5$O$_7$ · 2H$_2$O (ca. 6 g), ca. 5 g NaCl und 1N HCl oder 1N NaOH zur Einstellung eines pH-Wertes von 7,5, aufgefüllt mit Wasser für Injektionszwecke auf 1000 ml.) Die folgenden Arbeitsgänge werden dann unter septischen Bedingungen durchgeführt:

Im Dialysat wird der Titer eingestellt, danach wird D-Mannitose (20 g/l) darin gelöst. Der pH-Wert wird auf 7,5 mit 1N HCl oder 1N NaOH eingestellt, dann wird durch einen Membranfilter von 0,45 Micron Porengröße filtriert und die Lösung dann zur Lyophylisierung in die Spritzenkammer abgefüllt. Die Aktivität dieser Lösung beträgt 300-350 I.E./ml.

Beispiel 3

Herstellung einer konzentrierten Lösung von humanem antihemophilischen Faktor (AHF bzw. Faktor VIII).

AHF wird aus gepooltem menschlichen Plasma nach der Polyethylenglykol-Methode hergestellt. Das gefrorene Plasma wird bei kontrollierten Temperaturen aufgetaut und das Cryopräcipitat durch Zentrifugieren isoliert, in heparinisiertem destilliertem Wasser (80 Heparineinheiten pro ml) suspendiert und der pH-Wert der Lösung auf 7,0 mit verdünnter HCl eingestellt und dann bei 28°C unter vorsichtigem Mischen gut suspendiert. Das Volumen des heparinisierten destillierten Wassers beträgt ca. 3 l/kg Cryopräcipitat.

Dann wird Polyethylenglykol zu der AHF-Lösung bis zu einer Endkonzentration von 3% zugesetzt und bei 25°C gut eingemischt. Das pH der AHF-Suspension wird dann auf 6,3 mit verdünnter Essigsäure eingestellt. Die Suspension wird dann bei 25°C mindestens ca. 1/4 h weitergemischt. Dann wird zentrifugiert.

Die überstehende Flüssigkeit der Zentrifugation wird zur Entfernung aller festen Teilchen filtriert. Dann werden Tri-n-butylphosphat und Polysorbat 80 zur gefilterten AHF-Lösung auf eine Endkonzentration von 0,30% TNBP (Volumen/Gewicht) und 1% Polysorbat (Gewicht/Gewicht) zugesetzt. Das pH wird mit verdünnter Essigsäure auf 6,3 eingestellt und die Mischung bei 27°C über Nacht stehengelassen. Dann wird weiter PEG bis auf eine Endkonzentration von 12% zugesetzt. Die Suspension wird eine Zeit lang bei 25° wie oben gemischt und der gebildete AHF-Niederschlag dann abzentrifugiert.

Das PEG gefällte AHF wird bei 5° in 1,6 M Glycin-Lösung, die 0,23 M Citrat und 13 U/ml Heparin enthält, suspendiert. Das Volumen der Glycinlösung beträgt 20 l/kg gefälltes AHF. Wenn die Suspension zur Homogenität gemischt ist, wird sie zentrifugiert, wobei AHF-Niederschlag gewonnen wird.

Das Glycin gefällte AHF wird wieder in Glycinlösung bei 5°, die wieder Citrat und Heparin enthält, resuspendiert. Das Volumen der Glycinlösung beträgt 20 l/kg gefälltes AHF. Nach Mischen der Suspension zur Homogenität wird wieder zentrifugiert, um AHF-Niederschlag zu gewinnen.

Dieser Niederschlag wird in 0,4 M EDTA (pH 7,3-7,5) (8 l/kg) aufgenommen und gegen Waschpuffer (0,02 M Natriumcitrat + 0,05 M Natriumchlorid, pH 6,6 bis 7,0) dialysiert.

Dies kann mehrfach wiederholt werden, bis die gewünschte Konzentration erreicht ist.

Als Alternative kann dieser Niederschlag in Glycin-Natriumcitratlösung bei 20°C gelöst werden, wobei das pH mit verdünnter NaOH oder HCL auf 7,3 eingestellt wird. Das Volumen an Glycin-Natriumcitratlösung beträgt 20 l/kg Glycin gefälltes AHF. Diese Lösung wird dann durch vorher sterilisierte Bakterien zurückhaltende Membranfilter steril filtriert und unter aseptischen Bedingungen in die Spritzen abgefüllt und lyophylisiert. Die Konzentration beträgt ca. 300-350 I.E./ml.

Beispiel 4

Herstellung von Faktor IX

Das in üblicher Weise erhaltene Faktor IX-Komplex-Pulver (Prothrombin Komplex) wird in destilliertem Wasser in einer Proteinkonzentration von 1,2% gelöst. Dann wird mit Verdünnungspuffer (0,02 M Na-citrat + 0,25 M NaCl, pH 7,3 ; 4,0 l/l) versetzt und zu dieser Lösung werden 0,16 l/l 1 M-Bariumchloridlösung gegeben, um das Faktor IX Protein auszufällen. Das gefällte Protein wird abfiltriert oder abzentrifugiert und dann in einer Lösung von 0,4 M EDTA in einer Menge von 8 l/kg gelöst und mit Waschpuffer (0,02 M Na-citrat + 0,05 M NaCl) diafiltriert. Der diafiltrierte Faktor IX wird so oft auf eine Chromatographiesäule aufgezogen, die Dextransulfat-SiO$_2$Harz enthält, und eluiert, bis die gewünschte Konzentration erreicht ist. Die Menge an Harz in der Säule beträgt etwa 2,5 l/100g verarbeitetes Ausgangsmaterial.

Der am Harz adsorbierte Faktor IX wird in der Säule mit 10 oder mehr Volumen Waschpuffer gewaschen, der dann verworfen wird. Gereinigtes Faktor IX wird von Harz mit einem linearen Salzgradienten eluiert, indem ein Elutionspuffer aus 0,02 M Natriumcitrat und 0,5 M Natriumchlorid von pH 6,6 bis 7 allmählich in der Menge erhöht wird. Am Ende des Gradienten wird die Säule weiter mit zusätzlichem Elutionspuffer eluiert. Das Faktor IX Eluat wird gepoolt, filtriert und, ggfs. nach Diafiltration, auf pH 7 eingestellt, in die Spritzen abgefüllt und lyophylisiert. Die Konzentration der Lösung beträgt im Schnitt ca. 300-350 I.E./ml.

**Patentansprüche**

1. Zweikammerspritze mit pyrogenfreiem sterilen Lösungsmittel in der der Spritzennadel abgekehrten Kammer, dadurch gekennzeichnet, daß sie in der, der Spritzennadel zugekehrten zweiten Kammer eine durch einmaliges Befüllen und Lyophilisieren in der Spritze selbst erhaltene Füllung aus aktivitätsempfindlichem humanen Protein in einer zur therapeutisch wirksamen einmaligen Verabreichung erforderlichen Menge aufweist.

2. Zweikammerspritze nach Anspruch 1, dadurch gekennzeichnet, daß die Füllung aus Urokinase, Pro-Urokinase, TPA, Streptokinase, Faktor VIII, Faktor IX oder Antithrombin III besteht.

3. Verfahren zur Herstellung einer Spritze nach Anspruch 1 oder 2 durch Befüllen, dadurch gekennzeichnet, daß man eine hinreichend konzentrierte Lösung des aktivitätsempfindlichen humanen Proteins in die der Injektionskanüle zugekehrte zweite Kammer einer Zweikammerspritze einfüllt und in situ lyophilisiert.

4. Mittel zur Durchführung des Verfahrens nach Anspruch 3 in Form von konzentrierten Lösungen aktivitätsempfindlicher humaner Proteine, dadurch gekennzeichnet, daß sie erhalten sind durch Isolierung, wobei mit oder ohne PEG Vorreinigung so oft dialysiert und/oder chromatographiert wird, bis die gewünschte Konzentration erreicht ist.

5. Mittel nach Anspruch 4, dadurch gekennzeichnet, daß die Dialyse aus einer leicht basischen Lösung, vorzugsweise pH 7,3 bis 7,5, gegen einen leicht sauren Puffer, vorzugsweise pH 6,6 bis 7,0, und die Elution bei der Chromatographie mit einem leicht sauren Puffer, insbesondere pH 6,6 bis 7,0 durchgeführt wird.

6. Verwendung des Mittels nach Anspruch 4 oder 5 zu Gestellung von gefüllten Zweikammespritzen nach Anspruch 1 oder 2 zur Sofortanwendung von aktivitätsempfindlichen humanen Proteinen und zur Heimselbstbehandlung.

## Claims

1. Twin-chamber syringe with pyrogen-free, sterile solvent in the chamber averted from the needle, **characterized** by having in the second chamber, facing the needle, in a quantity required for a single, therapeutically effective application, a charge of activity-sensitive human protein introduced and lyophilized in a single operation in the syringe.

2. Twin-chamber syringe according to claim 1, **characterized** by the charge consisting of urokinase, pro-urokinase, TPA, streptokinase, factor VIII, factor IX, or antithrombin III.

3. Method for the production of a syringe according to claims 1 or 2 by charging said syringe, **characterized** by filling a sufficiently concentrated solution of the activity-sensitive, human protein into the second chamber, facing the needle, of a syringe, and lyophilizing in situ.

4. Means to carry out the method according to claim 3 in the form of concentrated solutions of activity-sensitive, human proteins, **characterized** by being obtained by a preparation method, while dialysing and/or chromatographing, with or without prepurification with PEG, as many times as is necessary to reach the desired concentration.

5. Means according to claim 4, **characterized** by the dialysis being performed from a slightly basic solution, preferably at pH 7.3 to 7.5, against a slightly acidic buffer, preferably at pH 6.6 to 7.0, and the elution in the chromatography being performed with a slightly acidic buffer, particularily at pH 6.6 to 7.0.

6. Use of the means according to claim 4 or 5 for preparing charged twin-chamber syringes according to claim 1 or 2 for the immediate application of activity-sensitive, human proteins and for self-administration at home.

## Revendications

1. Seringue à deux compartiments, contenant un solvant stérile exempt de pyrogène dans le compartiment éloigné de l'aiguille de la seringue, caractérisée en ce qu'elle contient dans le deuxième compartiment, adjacent à l'aiguille de la seringue, une charge de protéine humaine du type dit sensible, obtenue par un remplissage unique et une lyophilisation dans la seringue proprement dite, en une quantité nécessaire à une administration unique qui est efficace du point de vue thérapeutique.

2. Seringue à deux compartiments selon la revendication 1, caractérisée en ce que la charge est composée d'urokinase, de pro-urokinase, de TPA, de streptokinase, du facteur VIII, du facteur IX ou d'antithrombine III.

3. Procédé de préparation d'une seringue selon la revendication 1 ou 2, par remplissage, caractérisé en ce qu'on introduit une solution suffisamment concentrée de la protéine humaine sensible dans le deuxième compartiment, tourné vers la canule d'injection, d'une seringue à deux compartiments, et on la lyophilise in situ.

4. Agents pour la mise en oeuvre du procédé selon la revendication 3, sous forme de solutions concentrées de protéines humaines sensibles, caractérisés en ce qu'on les obtient par isolement, en effectuant une dialyse répétée, avec ou sans purification préalable avec du PEG, et/ou une chromatographie répétée, jusqu'à ce qu'on obtienne la concentration souhaitée.

5. Agents selon la revendication 4, caractérisés en ce qu'on réalise la dialyse à partir d'une solution légèrement basique, de préférence à pH 7,3 à 7,5, à travers un tampon légèrement acide, de préférence à pH 6,6 à 7,0, et l'élution de la chromatographie avec un tampon légèrement acide, en particulier à pH 6,6 à 7,0.

6. Utilisation de l'agent selon la revendication 4 ou 5, pour garnir des seringues remplies selon la revendication 1 ou 2 pour une application instantanée de protéines humaines sensibles et pour un traitement à domicile par le patient lui-même.